# EUROPEAN PATENT SPECIFICATION

(11) **EP 1 941 877 B1**
(45) Date of publication and mention of the grant of the patent: **18.11.2009**
(21) Application number: 06791280.8
(22) Date of filing: 18.09.2006
(51) Int. Cl.: A61K 31/34, A61K 33/00

(54) **PHARMACEUTICAL COMPOSITIONS CONTAINING NITROVINYLFURAN DERIVATIVES FOR THE TREATMENT OF LEISHMANIASIS AND TRYPANOSOMIASIS**
PHARMAZEUTISCHE ZUSAMMENSETZUNGEN MIT NITROVINYLFURAN-DERIVATEN ZUR BEHANDLUNG VON LEISHMANIASIS UND TRYPANOSOMIASIS
COMPOSITIONS PHARMACEUTIQUES CONTENANT DES DERIVES NITROVINYLFURANIQUES POUR LE TRAITEMENT DE LA LEISHMANIOSE ET DE LA TRYPANOSOMOSE

(30) Priority: 26.09.2005 CU 20050175
(43) Date of publication of application: 09.07.2008
(73) Proprietor: CENTRO DE BIOACTIVOS QUIMICOS, Santa Clara, Villa Clara 54830 (CU)
(72) Inventor: CASTAÑEDO CANCIO, Nilo, Ramón, Villa Clara 50100 (CU); SIFONTES RODRIGUEZ, Sergio, Ciudad De La Habana 11500 (CU); MONZOTE FIDALGO, Lianet, Ciudad De La Habana 13900 (CU); LOPEZ HERNANDEZ, Yamilé, Ciudad De La Habana 11500 (CU); MONTALVO ÁLVAREZ, Ana, Margarita, Ciudad De La Habana 10500 (CU); INFANTE BOURZAC, Juan, Francisco, Ciudad De La Habana 10300 (CU); OLAZABAL MANSO, Ervelio, Eliseo, Villa Clara 50300 (CU)
(74) Representative: Hatzmann, Martin
(86) International application number: PCT/CU2006/000009
(87) International publication number: WO 2007/033616

(56) References cited:
- EP-A1- 1 249 449
- US-A- 5 416 107
- BUEDING E ET AL: "THE ANTISCHISTOSOMAL ACTIVITY OF A NITROVINYLFURAN DERIVATIVE (SQ 18,506) IN MICE AND HAMSTERS" JOURNAL OF PHARMACOLOGY AND EXPERIMENTAL THERAPEUTICS, AMERICAN SOCIETY FOR PHARMACOLOGY AND, US, vol. 178, no. 2, August 1971 (1971-08), pages 402-410, XP009076514 ISSN: 0022-3565

## Description

### Field of the invention

The present invention regards to human and animal health and, in general has to do with a family of chemical compounds biologically active against the etiological agents of leishmaniosis and trypanosomosis.

### Previous Art

Leishmaniosis is a disease caused by protozoan parasites of the genus *Leishmania.* This genus comprises about 20 pathogenic species for human beings and animals. The disease has been described in 88 countries and 1.5-2 million new cases are reported yearly. It is estimated that 12 million people are affected by the disease worldwide and 350 million are at risk every year (CDC. http://www.dpd.cdc.cdc./dpdx., Parasitic Disease Information. Fact Sheet 1. April 2004).

There are three clinical forms:
Cutaneous leishmaniosis: The cutaneous form is the commonest one, accounting for 90% of the cases. Cutaneous ulcers that take from moths to years to heal are typical. Secondary bacterial or fungal infections frequently worsen the primary lesion.
Visceral leishmaniosis: The liver and spleen are enlarged, the bone marrow is damaged, and the patient suffers from anemia and progressive weight loss. Most untreated cases and 15-25 % of those who receive treatment in a late stage die.
Mucocutaneous leishmaniosis: Months to years after the primary cutaneous lesion healing has occurred, or even when the skin ulcer is still present, the parasites spread to the oral, nasal and pharyngeal mucosa. Soft tissues, mainly cartilages, are damaged, causing monstrous deformities of the face and eventually death due to breathing impairment once the laryngeal cartilages are destroyed.

The acquired immunodeficiency syndrome (AIDS) has aggravated the epidemiological situation, as patients require longer treatments and the frequency of relapses is higher (Alvar J, et .al. Clin Microbiol Rev 1997(10):298-319.) Pentavalent antimony derivatives have been the first line drugs for over 50 years. Nowadays, Pentostam^{®} (Sodium stibogluconate) and Glucantime^{®} (meglumine antimoniate) are the pharmaceutical forms in medical use. They are not effective against all species; being *L aethiopica* and *L. major* being the least sensible. Moreover, an increasing number of strains from traditionally sensitive species develop resistance. (Aparicio P, et. al. Terapéutica antiparasitaria, Enferm Infecc Microbiol Clin 2003; 21 (10):579-94). Side effects related to pentavalent antimony derivatives have been a frequent cause of treatment discontinuation. Subclinical pancreatitis (31%), cardiotoxicity (15%) and nephrotoxicity (5%) are the major side effects. AIDS patients co-infected, with *Leishmania* are notably sensitive to pancreatitis (Alvar J, et. al. Clin Microbiol Rev 1997 (10): 298-319.) Amphotericin B and pentamidine are the second line drugs for the treatment of leishmaniosis. They are reserved for pentavalent-antimony-resistant cases. Two less toxic, and effective Amphotericin B lipid formulations (Alphocil^{®} and AmBisone^{®}) have been developed, but the majority of the affected population cannot afford to pay for the treatment. (Aparicio P, et. al. Terapéutica antiparasitaria, Enferm Infecc Microbiol Clin 2003;21 (10):579-94).

The American trypanosomosis, also known as Chagas' disease, is an endemic health problem in Latin America caused by *Trypanosoma cruzi* infection. Around 50 000 people die every year as a consequence. Twenty million are estimated to suffer from this disease and other 100 millions are in danger of getting sick yearly. The contaminated feces of hematophagous insects are responsible for transmitting the parasite to people. (Anonymous, hftp://www.unl.edu.ar/eje.php?ID=1834, September 15 2005).

Hundreds of structurally different compounds have been tested against *T. cruzi* during the last six decades; however, just a few of them have passed the preclinical stage with relative success. Only two trypanocidal drugs have been licensed in Latin America: nifurtimox (1972-1992), which has been eventually withdrawn from the market and benznidazole, clinically available since 1975 (Paulino A, et al. Mini Rev Med Chem 2005;5(5):499-519; Lockman JW, Hamilton AD Curr Med Chem 2005;12(8):945-59).

### Description of the Invention

The present invention describes the action of pharmaceutical compositions containing any of the six following representatives of the β-nitrovinylfuran chemical family:
2-(2-nitrovinyl)-furan
2-bromo-5-(2-nitrovinyl)-furan
2-(2-bromo-2-nitrovinyl)-furan
2-bromo-5-(2-bromo-2-nitrovinyl)-furan,
2-(2-methyl-2-nitrovinyl)-furan
2-bromo-5-(2-methyl-2-nitrovinyl)-furan

Besides the β-nitrovinylfuran derivatives, these pharmaceutical compositions contain vehicles, excipients, solvents and adjuvants of pharmaceutical use. Solvents are preferably apolar ones, such as sunflower oil, petrolatum or Mygliol 810. These pharmaceutical compositions contain β-nitrovinytfuran derivatives at concentrations ranging from of 0.01 to 10%. The exact composition depends on the particular compound and the route of administration. The choice of a specific administration route, either topical or systemic, is determined by the clinical form of the disease. Trypanosomosis as well as mucocutaneous and visceral leishmaniosis are treated by routes that ensure a systemic distribution of the drug. On the contrary, cutaneous leishmaniosis and those cutaneous manifestations of the above-mentioned clinical forms of leishmaniosis are treated by both topical and systemic routes.

The activity of compounds was tested against three *Leishmania* species, which were representative of species causing the cutaneous, mucocutaneous and visceral forms of leishmaniosis. They were also tested against *Trypanosoma cruzi,* the etiological agent of Chagas' disease.

### Description of the figures

**Figure 1****:** *In vivo* activity of 2-bromo-5-(2-bromo-2-nitrovinyl)-furan and 2-bromo-5-(2-methyl-2-nitrovinyl)-furan against experimental cutaneous leishmaniosis. Early stage lesions treated every 24 h.

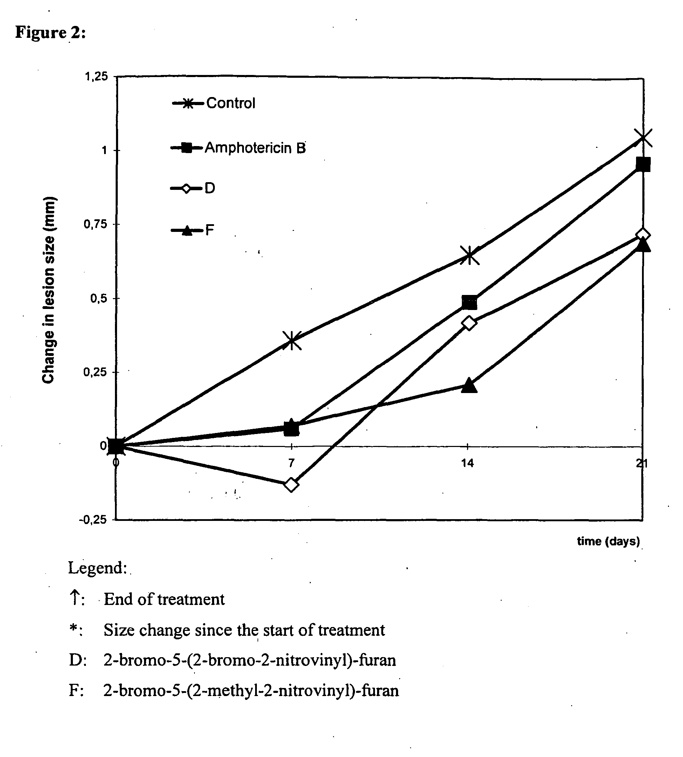

**Figure 2****:** *In vivo* activity of 2-bromo-5-(2-bromo-2-nitrovinyl)-furan and 2-bromo-5-(2-methyl-2-nitrovinyl)-furan against experimental cutaneous leishmaniosis. Early stage lesions treated every 12 h.
**Figure 3****:** *In vivo* activity of 2-bromo-5-(2-bromo-2-nitrovinyl)-furan and 2-bromo-5-(2-methyl-2-nitrovinyl)-furan against chronic experimental cutaneous leishmaniosis. Effect during the first week of treatment.
**Figure 4****:** *In vivo* activity of 2-bromo-5-(2-bromo-2-nitrovinyl)-furan and 2-bromo-5-(2-methyl-2-nitrovinyl)-furan against chronic experimental cutaneous leishmaniosis as measured by changes in lesion size from the beginning to the end of therapy.

### EXAMPLES FOR REALIZATION

### Example 1: In vitro activity of β-nitrovinylfuran derivatives against Leishmania promastigotes

Mean inhibitory concentrations (CI50%) against L. *mexicana amazonensis, L. donovani infantum* and L. *braziliensis braziliensis* promastigotes were determined according to the method described by Bodley AL et al (J Infect Dis 1995; 172(4):1157-9.). Minimal parasiticidal concentrations (CP100, minimal concentrations capable of abolishing the motility of all parasites in the culture) were also determined. Amphotericin B and Glucantime^{®} were tested as reference drugs.

All the β-nitrovinylfuran derivatives inhibited parasite growth at considerably low concentrations. Similarly, the compounds produced parasite death at low concentrations during the first 2-3 h following their addition to the culture media. Amphotericin B was more active than the β-nitrovinylfuran derivatives, but Glucantime^{®} was significantly less active.

**Table 1: In vitro activity of β-nitrovinylfuran derivatives against L. m. amazonensis, L. d. infantum and L. b. braziliensis promastigotes**

| **Compound** | *amazonensis* | | *braziliensis* | | *Infantum* | |
|---|---|---|---|---|---|---|
| | CI50 | CP100 | C150 | CP100 | C150 | CP100 |
| A | 0,66 | 1,12 | 0,20 | 1,29 | 0,35 | 1,75 |
| B | 0,45 | 2,08 | 0,18 | 0,61 | 0,23 | 0,51 |
| C | 0,63 | 1,77 | 0,25 | 1,25 | 0,36 | 2,01 |
| D | 0,25 | 1,53 | 0,26 | 0,49 | 0,35 | 0,86 |
| E | 0,34 | 3,49 | 0,35 | 4,84 | 0,31 | 1,27 |
| F | 0,20 | 1,42 | 0,24 | 1,32 | 0,20 | 0,62 |
| Amphotericin B | 0,027 | 0,051 | 0,013 | 0,028 | 0,013 | 0,016 |
| Glucantime^{®} | 1916 | >4250 | 1893 | >4250 | 1925 | >4250 |

| | | | | | | |
|---|---|---|---|---|---|---|
| Legend: A: 2-(2-nitrovinyl)-furan B: 2-bromo-5-(2-nitrovinyl)-furan C: 2-(2-bromo-2-nitrovinyl)-furan D: 2-bromo-5-(2-bromo-2-nitrovinyl)-furan E: 2-(2-methyl-2-nitrovinyl)-furan F: 2-bromo-5-(2-methyl-2-nitrovinyl)-furan | | | | | | |

### Example 2: In vitro activity against Trypanosoma cruzi

The *in vitro* activity of the β-nitrovinylfuran derivatives was tested against trypomastigotes of *T. cruzi* grown in a rat myoblast cell line. Minimal parasiticidal concentrations (minimal concentration that immobilizes all the parasites in the culture) and mean inhibitory concentrations (concentration that reduces the number of parasites to 50% of those found in the un-treated control cultures) were determined according to Buckner's method (Antimicrob Agents Chemother 1996;40(11):2592-7). The experiment evidenced that the test compounds had an *in vitro* parasiticidal activity superior to that of benznidazole.

**Table 2: In vitro effect β-nitrovinylfuran derivatives against T. cruzi**

| 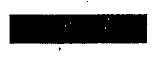 | CI50 | | CP100 | |
|---|---|---|---|---|
| | µg/mL | µM | µg/mL | µM |
| A | 1,8 | 12,6 | 10,0 | 71,9 |
| B | 1,2 | 5,7 | 3,3 | 15,3 |
| C | 3,7 | 16,9 | 10,0 | 45,9 |
| D | 4,1 | 13,9 | 10,0 | 33,7 |
| E | 4,7 | 31,0 | 30,0 | 196,1 |
| F | 5,3 | 23,0 | 30,0 | 129,3 |
| Benznidazole | 0,9 | 3,3 | >40 | >154 |

| | | | | |
|---|---|---|---|---|
| Legend: A: 2-(2-nitrovinyl)-furan B: 2-bromo-5-(2-nitrovinyl)-furan C: 2-(2-bromo-2-nitrovinyl)-furan D: 2-bromo-5-(2-bromo-2-nitrovinyl)-furan E: 2-(2-methyl-2-nitrovinyl)-furan F: 2-bromo-5-(2-methyl-2-nitrovinyl)-furan | | | | |

### Example 3: Toxicological studies

The same animal model used for *in vivo* anti-leishmanial tests was used to perform comparative toxicological studies of the β-nitrovinylfuran derivatives. The main excipient of the compositions used with this purpose was the sunflower oil. The mean lethal doses (LD50) of each β-nitrovinylfuran derivative and Amphotericin B were estimated in female Balb/c mice (18-20g body weight), treated by a single intraperitoneal injection. The maximal non-lethal doses (LD0) were calculated from the dose-mortality curves. The maximal tolerated doses (MTD, maximal dose causing neither -mortality nor body weight losses over 10% respect to the initial weight) by repeated intraperitoneal administration every 12 or 24 h during 14 days were determined as well. Results indicated that the β-nitrovinylfuran derivatives had a relatively wide range of LD50's, which were all superior to that of Amphotericin B. The MTD determined during the course of the present series of experiments allowed choosing a proper dose for *in vivo* anti-leishmanial activity studies.

**Table 3: Mean lethal doses (LD50), Maximal non-lethal doses (LD0) and Maximal tolerated doses MTD) of β-nitrovinylfuran derivatives and Amphotericin B.**

| Compound | LD50 (mg/Kg) | LD0 (mg/Kg) | MTD (every 24h) | MTD (every 12h) |
|---|---|---|---|---|
| A | 68 | 50 | 55 | - |
| B | 73 | 45 | 20 | - |
| C | 51 | 35 | - | - |
| D | 24 | 15 | 5 | 2-3 |
| E | 80 | 65 | 60 | - |
| F | 246 | 200 | 100 | 100 |
| Amphotericin B | 22 | 15 | - | - |

| | | | | |
|---|---|---|---|---|
| Legend: A: 2-(2-nitrovinyl)-furan B: 2-bromo-5-(2-nitrovinyl)-furan C: 2-(2-bromo-2-nitrovinyl)-furan D: 2-bromo-5-(2-bromo-2-nitrovinyl)-furan E: 2-(2-methyl-2-nitrovinyl)-furan F: 2-bromo-5-(2-methyl-2-nitrovinyl)-furan | | | | |

### Example 4: Treatment of experimental cutaneous leishmaniosis with 2-bromo-5-(2-bromo-2-nitrovinyl)-furan and 2-bromo-5-(2-methyl-2-nitrovinyl)-furan. Early stage lesions treated every 24 h.

Balb/c mice were experimentally infected with *L. m. amazonensis* promastigotes by intradermal injection in the footpads and six weeks later they were treated with the test compounds by intraperitoneal route. The product 2-bromo-5-(2-bromo-2-nitrovinyl)-furan was administered at a dose of 5 mg/Kg; the 2-bromo-5-(2-methyl-2-nitrovinyl)-furan, at 50 mg/Kg and Amphotericin B, at 1 mg/Kg. Animals were daily dosed for 14 days. Miglyol 810, the vehicle used for the β-nitrovinylfuran derivatives, was similarly administered to a group of mice. A group of infected mice that did not receive any treatment at all was also included as control.

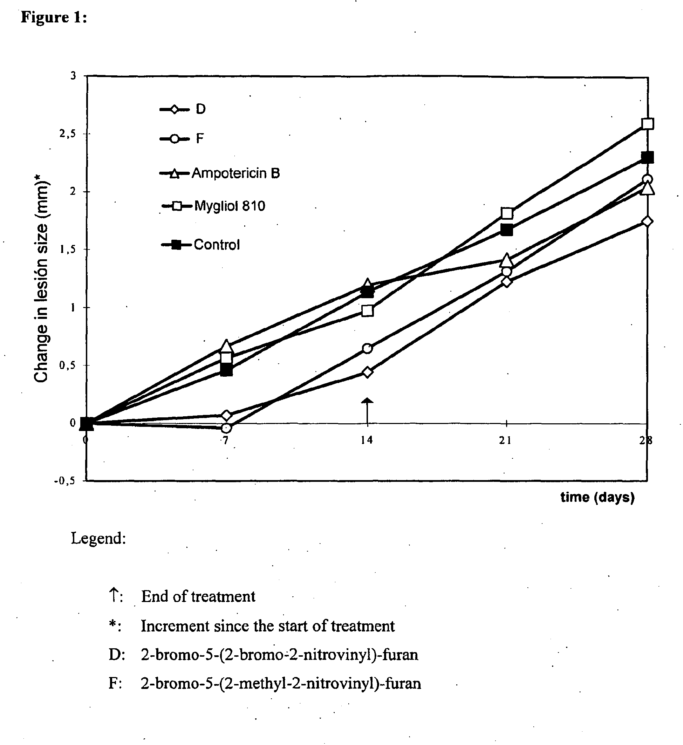

The lesion growth curves (Figure 1) evidenced that mice treated with either 2-bromo-5-(2-bromo-2-nitrovinyl)-furan or 2-bromo-5-(2-methyl-2-nitrovinyl)-furan had a minimal lesion growth during the first week of treatment. Those animals treated with 2-bromo-5-(2-methyl-2-nitrovinyl)-furan showed a small decrease in lesion size. Lesion growth during that week was statistically lower on the test group compared to the rest of the groups. During the second week of treatment the growth rate was comparable in all the experimental groups. However, the differences established during the first week of treatment with 2-bromo-5-(2-bromo-2-nitrovinyl)-furan remained for two other weeks after treatment had finished. The group of mice treated with Amphotericin B evolved similarly to controls; however, during the first week after discontinuation of treatment they showed a remarkable decrease of lesion growth rate.

### Example 5: Treatment of experimental cutaneous leishmaniosis with 2-bromo-5-(2-bromo-2-nitrovinyl)-furan and 2-bromo-5-(2-methyl-2-nitrovinyl)-furan. Early stage lesions treated every 12 h.

The experiment was essentially carried out in a similar way as the one described above. However, 2-bromo-5-(2-bromo-2-nitrovinyl)-furan was administered at a dose of 2 mg/Kg and 2-bromo-5-(2-methyl-2-nitrovinyl)-furan at 100 mg/Kg. Treatments were administered by intraperitoneal route, but every 12 h instead of 24 h.

The lesions of mice treated with 5-(2-bromo-2-nitrovinyl)-furan decreased during the first week of treatment ( 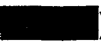 ), been statistically smaller (P<0.001) than those of control mice. The following week, lesions grew at a similar rate as control and the next week, a decline in lesion growth rate was then noticed. Seven days after finishing treatment, mice treated with 5-(2-bromo-2-nitrovinyl)-furan had lesions statistically (P<0.05) smaller than those of controls.

Lesion growth rate of mice treated with either 2-bromo-5-(2-methyl-2-nitrovinyl)-furan or 2-bromo-5-(2-bromo-2-nitrovinyl)-furan was significantly lower compared to controls. Similarly, both products showed higher activity than Amphotericin B.

### Example 6: Treatment of experimental cutaneous leishmaniosis with 2-bromo-5-(2-bromo-2-nitrovinyl)-furan and 2-bromo-5-(2-methyl-2-nitrovinyl)-furan. Chronic lesions treated every 12 h.

Mice developed chronic leishmanial lesions at eighteen weeks post-infection. Lesions were ulcerated and cowered with a thick crust. The average dorso-plantar diameter of the infected foot was 1.23 ± 0.24 cm and the lateral diameter was 1.24 ± 0.19 cm. At that stage the treatment started twice a day with either 2-bromo-5-(2-bromo-2-nitrovihyl)-furan at 2 mg/Kg; 2-bromo-5-(2-methyl-2-nitrovinyl)-furan at 50 mg/Kg or Amphotericin B at 5 mg/Kg every other day. A control group formed by infected non-treated mice was also included in the experiment. All drugs were administered by intraperitoneal route for 14 days.

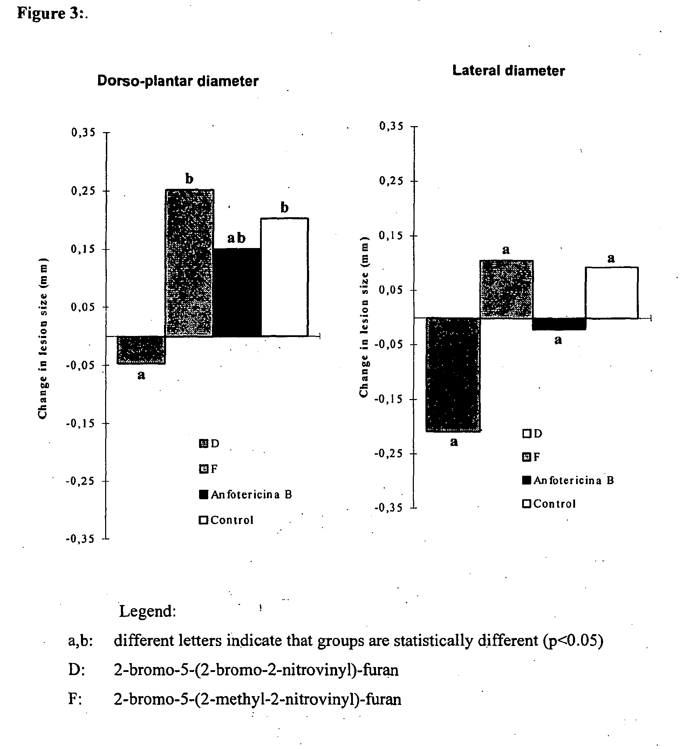

During the first week of treatment with 2-bromo-5-(2-bromo-2-nitrovinyl)-furan mice showed a decrease of the dorso-plantar diameter (Figure 3) of the infected foot that differed from the control group (P<0.05). Although lateral diameter of the lesions of these mice also revealed a decrease, it was comparable to the diameter of control mice (P>0.05). The 2-bromo-5-(2-methyl-2-nitrovinyl)-furan did not prove to be effective for the treatment of chronic lesions. Amphotericin B was not effective, since neither the dorso-plantar nor the lateral diameters differed statistically from the lesions of the control group (P>0.05).

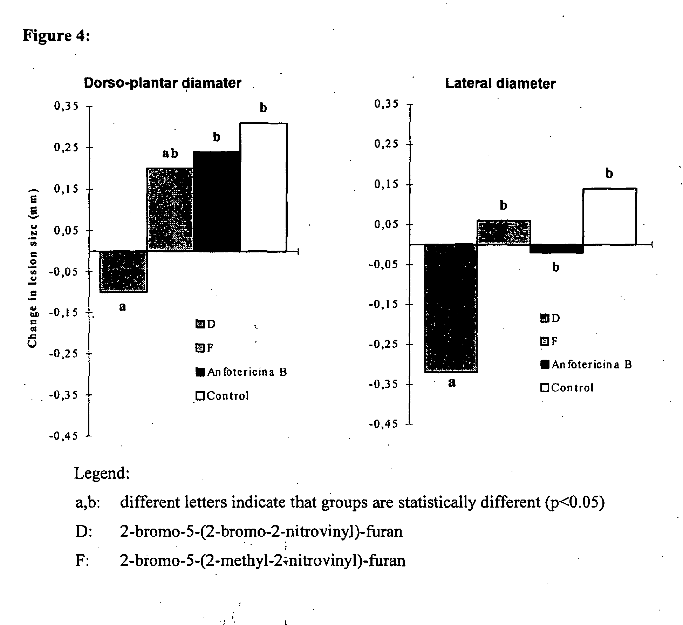

By the end of treatment (Figure 4), mice treated with 2-bromo-5-(2-bromo-2-nitrovinyl)-furan evidenced a decrease of lesion size. Lesions of these mice were significantly different to the ones of control mice in both the dorso-plantar (P<0.05) and lateral (P<0.01) diameters. These mice also differed from those treated with Amphotericin B (P<0.05). On the contrary, treatment with 2-bromo-5-(2-methyl-2-nitrovinyl)-furan was ineffective.

Animals treated with Amphotericin B incremented their lesion dorso-plantar diameters as controls (P>0.05). Although the lateral diameter diminished in the lesions of Amphotericin B-treated mice, the change was not different (P>0.05) to that found in controls.

The graphic illustrates that control, mice incremented their lesions mainly by the dorso-plantar diameter. Interestingly, the therapeutic effect of the test compounds is more evident in relation to the lateral diameter.

The present study showed that 2-bromo-5-(2-bromo-2-nitrovinyl)-furan has *in vivo* activity against *Leishmania,* as demonstrated in the biomodel of experimental cutaneous leishmaniosis developed in Balb/c mice inoculated with L. m. *amazonensis.* On the other hand, although higher doses of the compound 2-bromo-5-(2-methyl-2-nitrovinyl)-furan are needed, an *in vivo* effect against the experimental disease was also showed.

### Example 7: Treatment of human cutaneous leishmaniosis with 2-bromo-5-(2-bromo-2-nitrovinyl)-furan

One hundred patients with parasitological diagnose of cutaneous leishmaniosis were treated. Patients were topically treated, once a day, for 21 days with an oily ointment containing 0.15% 2-bromo-5-(2-bromo-2-nitrovinyl)-furan as active ingredient and petrolatum as the main excipient.

Ninety percent of patients showed total cure of the cutaneous lesions. The time of recovery depended on the severity of lesions and varied from 10 to 21 days. At the end of the treatment period, the 90% of patients that had recovered evidenced not only the cure of the ulcers, but the local and systemic symptoms due to the primary effect of the parasite infection and the secondary bacterial or fungal contaminants had disappeared as well.

Patients did not evidence any side effect that impeded the continuation of therapy as conceived in the protocol.

## Claims

1. Pharmaceutical compositions containing nitrovinylfuran derivatives for the treatment of leishmaniosis and tripanosomosis.

2. Pharmaceutical compositions according to claim 1 considering that leishmaniosis comprises the cutaneous, mucocutaneous and visceral clinical forms and tripanosomosis refers to that caused by *Trypanosoma cruzi.*

3. Pharmaceutical compositions according to claims 1 and 2 considering that nitrovinylfuran derivatives are: 2-(2-nitrovinyl)-furan, 2-bromo-5-(2-nitrovinyl)-furan, 2-(2-bromo-2-nitrovinyl)-furan, 2-bromo-5-(2-bromo-2-nitrovinyl)-furan, 2-(2-methyl-2-nitrovinyl)-furan or 2-bromo-5-(2-methyl-2-nitrovinyl)-furan.

4. Pharmaceutical compositions according to claims 1 to 3 considering that the routes of administration are either topical or systemic.

5. Pharmaceutical compositions according to claims 1 to 4 containing the pharmaceutical active ingredient and, depending on the route of administration, oily /ointments, oils and glycols as well.

6. Pharmaceutical compositions according to claims 1 to 5 which are adequate for the combined treatment of leishmaniosis and opportunistic bacterial and fungal diseases associated with the leishmanial lesions.

## Patentansprüche

1. Pharmazeutische Zusammensetzung enthaltend Nitrovinylfuranderivate für die Behandlung von Leishmaniose und Trypanosomiasis.

2. Pharmazeutische Zusammensetzung nach Anspruch 1, **dadurch gekennzeichnet, dass** Leishmaniose die kutanen, mukokutanen und viszeralen klinischen Formen umfasst und Trypanosomiasis sich auf die durch *Trypanosoma cruzi* verursachte Trypanosomiasis bezieht.

3. Pharmazeutische Zusammensetzung nach den Ansprüchen 1 und 2, **dadurch gekennzeichnet, dass** die Nitrovinylfuranderivate 2-(2-Nitrovinyl)-furan, 2-Brom-5-(2-nitrovinyl)-furan, 2-(2-Brom-2-nitrovinyl)-furan, 2-Brom-5-(2-brom-2-nitrovinyl)-furan, 2-(2-Methyl-2-nitrovinyl)-furan oder 2-Brom-5-(2-methyl-2-nitrovinyl)-furan sind.

4. Pharmazeutische Zusammensetzung nach den Ansprüchen 1 bis 3, **dadurch gekennzeichnet, dass** die Verabreichungswege entweder topisch oder systemisch sind.

5. Pharmazeutische Zusammensetzung nach den Ansprüchen 1 bis 4, enthaltend den pharmazeutischen Wirkstoff und, in Abhängigkeit von dem Verabreichungsweg, ölige bzw. ölhaltige Salben, Öle sowie Glycole.

6. Pharmazeutische Zusammensetzung nach den Ansprüchen 1 bis 5, welche für die kombinierte Behandlung von Leishmaniose und opportunistischen Bakterien- und Pilzerkrankungen geeignet ist, die mit den Leishmaniose-Läsionen verbunden sind.

## Revendications

1. Compositions pharmaceutiques contenant des dérivés de nitrovinylfuranne pour le traitement de la leishmaniose et de la trypanosomose.

2. Compositions pharmaceutiques selon la revendication 1, dans lesquelles la leishmaniose comprend les formes cliniques cutanée, mucocutanée et viscérale et que la trypanosomose désigne la trypanosomose provoquée par *Trypanosome cruzi.*

3. Compositions pharmaceutiques selon les revendications 1 et 2, dans lesquelles les dérivés de nitrovinylfuranne sont les composés suivants : 2-(2-nitrovinyl)-furanne, 2-bromo-5-(2-nitrovinyl)-furanne, 2-(2-bromo-2-nitrovinyl)-furanne, 2-bromo-5-(2-bromo-2-nitrovinyl)-furanne, 2-(2-méthyl-2-nitrovinyl)-furanne ou 2-bromo-5-(2-méthyl-2-nitrovinyl)-furanne.

4. Compositions pharmaceutiques selon les revendications 1 à 3, dans lesquelles les voies d'administration sont topiques ou systémiques.

5. Compositions pharmaceutiques selon les revendications 1 à 4, contenant l'ingrédient pharmaceutique actif, ainsi que, en fonction de la voie d'administration, des onguents huileux, des huiles et des glycols.

6. Compositions pharmaceutiques selon les revendications 1 à 5, lesquelles sont appropriées pour le traitement combiné de la leishmaniose et de maladies bactériennes et fongiques opportunistes associées aux lésions leishmaniennes.
